# EUROPEAN PATENT SPECIFICATION

(11) **EP 3 377 089 B1**
(45) Date of publication and mention of the grant of the patent: **08.04.2020**
(21) Application number: 16809895.2
(22) Date of filing: 18.11.2016
(51) Int. Cl.: A61K 38/18, A61P 25/00, A61P 25/16, A61P 25/28

(54) **COMPOSITIONS COMPRISING CDNF FOR USE IN THE INTRANASAL TREATMENT OF CENTRAL NERVOUS SYSTEM DISEASES**
ZUSAMMENSETZUNGEN MIT CDNF ZUR VERWENDUNG BEI DER INTRANASALEN BEHANDLUNG VON ERKRANKUNGEN DES ZENTRALNERVENSYSTEMS
COMPOSITIONS COMPRENANT DU CDNF POUR LE TRAITEMENT PAR VOIE INTRANASALE DE MALADIES DU SYSTÈME NERVEUX CENTRAL

(30) Priority: 18.11.2015 FI 20155857
(43) Date of publication of application: 26.09.2018
(73) Proprietor: Herantis Pharma Plc, 02600 Espoo (FI)
(72) Inventor: HUTTUNEN, Henri, 00670 Helsinki (FI); AIRAVAARA, Mikko, 00730 Helsinki (FI); SAARMA, Mart, 00570 Helsinki (FI)
(74) Representative: Laine IP Oy
(86) International application number: PCT/FI2016/050813
(87) International publication number: WO 2017/085362

(56) References cited:
- WO-A1-2014/066686
- WO-A2-02/074956
- VOUTILAINEN MERJA H ET AL: "Therapeutic potential of the endoplasmic reticulum located and secreted CDNF/MANF family of neurotrophic factors in Parkinson's disease", FEBS LETTERS, vol. 589, no. 24, 9 October 2015 (2015-10-09), pages 3739-3748, XP029326379, ISSN: 0014-5793, DOI: 10.1016/J.FEBSLET.2015.09.031
- BENDER T S ET AL: "Intranasal administration of glial-derived neurotrophic factor (GDNF) rapidly and significantly increases whole-brain GDNF level in rats", NEUROSCIENCE, vol. 303, 10 September 2015 (2015-09-10), pages 569-576, XP029254430, ISSN: 0306-4522, DOI: 10.1016/J.NEUROSCIENCE.2015.07.016
- SANDRA R. ALCALÁ-BARRAZA ET AL: "Intranasal delivery of neurotrophic factors BDNF, CNTF, EPO, and NT-4 to the CNS", JOURNAL OF DRUG TARGETING, vol. 18, no. 3, 6 October 2009 (2009-10-06), pages 179-190, XP55321545, ISSN: 1061-186X, DOI: 10.3109/10611860903318134
- ALY AE ET AL: "Intranasal gene delivery for treating Parkinson's disease: overcoming the blood-brain barrier", EXPERT OPINION ON DRUG DELIVERY, INFORMA HEALTHCARE, GB, vol. 18, 1 August 2015 (2015-08-01), pages 1-19, XP009186474, ISSN: 1742-5247, DOI: 10.1517/17425247.2015.1069815

### SEQUENCE LISTING

<110> Herantis Pharma plc
<120> Compositions comprising CDNF or MANF for use in the intranasal treatment of central nervous system diseases
<130> 53791
<150> FI 20155857
   <151> 2015-11-18
<160> 6
<170> PatentIn version 3.1
<210> 1
   <211> 161
   <212> PRT
   <213> Homo sapiens
<400> 1
   Gln Glu Ala Gly Gly Arg Pro Gly Ala Asp Cys Glu Val Cys Lys Glu
   1               5                   10                  15
   Phe Leu Asn Arg Phe Tyr Lys Ser Leu Ile Asp Arg Gly Val Asn Phe
               20                  25                  30
   Ser Leu Asp Thr Ile Glu Lys Glu Leu Ile Ser Phe Cys Leu Asp Thr
           35                  40                  45
   Lys Gly Lys Glu Asn Arg Leu Cys Tyr Tyr Leu Gly Ala Thr Lys Asp
       50                  55                  60
   Ala Ala Thr Lys Ile Leu Ser Glu Val Thr Arg Pro Met Ser Val His
   65                  70                  75                  80
   Met Pro Ala Met Lys Ile Cys Glu Lys Leu Lys Lys Leu Asp Ser Gln
                   85                  90                  95
   Ile Cys Glu Leu Lys Tyr Glu Lys Thr Leu Asp Leu Ala Ser Val Asp
               100                 105                 110
   Leu Arg Lys Met Arg Val Ala Glu Leu Lys Gln Ile Leu His Ser Trp
           115                 120                 125
   Gly Glu Glu Cys Arg Ala Cys Ala Glu Lys Thr Asp Tyr Val Asn Leu
       130                 135                 140
   Ile Gln Glu Leu Ala Pro Lys Tyr Ala Ala Thr His Pro Lys Thr Glu
   145                 150                 155                 160
   Leu
<210> 2
   <211> 187
   <212> PRT
   <213> Homo sapiens
<400> 2
   Met Trp Cys Ala Ser Pro Val Ala Val Val Ala Phe Cys Ala Gly Leu
   1               5                   10                  15
   Leu Val Ser His Pro Val Leu Thr Gln Gly Gln Glu Ala Gly Gly Arg
               20                  25                  30
   Pro Gly Ala Asp Cys Glu Val Cys Lys Glu Phe Leu Asn Arg Phe Tyr
           35                  40                  45
   Lys Ser Leu Ile Asp Arg Gly Val Asn Phe Ser Leu Asp Thr Ile Glu
       50                  55                  60
   Lys Glu Leu Ile Ser Phe Cys Leu Asp Thr Lys Gly Lys Glu Asn Arg
   65                  70                  75                  80
   Leu Cys Tyr Tyr Leu Gly Ala Thr Lys Asp Ala Ala Thr Lys Ile Leu
                   85                  90                  95
   Ser Glu Val Thr Arg Pro Met Ser Val His Met Pro Ala Met Lys Ile
               100                 105                 110
   Cys Glu Lys Leu Lys Lys Leu Asp Ser Gln Ile Cys Glu Leu Lys Tyr
           115                 120                 125
   Glu Lys Thr Leu Asp Leu Ala Ser Val Asp Leu Arg Lys Met Arg Val
       130                 135                 140
   Ala Glu Leu Lys Gln Ile Leu His Ser Trp Gly Glu Glu Cys Arg Ala
   145                 150                 155                 160
   Cys Ala Glu Lys Thr Asp Tyr Val Asn Leu Ile Gln Glu Leu Ala Pro
                   165                 170                 175
   Lys Tyr Ala Ala Thr His Pro Lys Thr Glu Leu
               180                 185
<210> 3
   <211> 158
   <212> PRT
   <213> Homo sapiens
<400> 3
   Leu Arg Pro Gly Asp Cys Glu Val Cys Ile Ser Tyr Leu Gly Arg Phe
   1               5                   10                  15
   Tyr Gln Asp Leu Lys Asp Arg Asp Val Thr Phe Ser Pro Ala Thr Ile
               20                  25                  30
   Glu Asn Glu Leu Ile Lys Phe Cys Arg Glu Ala Arg Gly Lys Glu Asn
           35                  40                  45
   Arg Leu Cys Tyr Tyr Ile Gly Ala Thr Asp Asp Ala Ala Thr Lys Ile
       50                  55                  60
   Ile Asn Glu Val Ser Lys Pro Leu Ala His His Ile Pro Val Glu Lys
   65                  70                  75                  80
   Ile Cys Glu Lys Leu Lys Lys Lys Asp Ser Gln Ile Cys Glu Leu Lys
                   85                  90                  95
   Tyr Asp Lys Gln Ile Asp Leu Ser Thr Val Asp Leu Lys Lys Leu Arg
               100                 105                 110
   Val Lys Glu Leu Lys Lys Ile Leu Asp Asp Trp Gly Glu Thr Cys Lys
           115                 120                 125
   Gly Cys Ala Glu Lys Ser Asp Tyr Ile Arg Lys Ile Asn Glu Leu Met
       130                 135                 140
   Pro Lys Tyr Ala Pro Lys Ala Ala Ser Ala Arg Thr Asp Leu
   145                 150                 155
<210> 4
   <211> 179
   <212> PRT
   <213> Homo sapiens
<400> 4
<210> 5
   <211> 4
   <212> PRT
   <213> Homo sapiens
<400> 5
   Cys Lys Gly Cys
   1
<210> 6
   <211> 4
   <212> PRT
   <213> Homo sapiens
<400> 6
   Cys Arg Ala Cys
   1

## Description

### TECHNICAL FIELD

The present disclosure relates to the field of neurotrophic factors and endoplasmic reticulum (ER) located proteins and more particularly to the field of treating central nervous system diseases. In particular, the present invention is directed to a composition suitable for intranasal administration for use in the intranasal treatment of a central nervous system disease, such as Alzheimer's disease, Parkinson's disease, multiple system atrophy, amyotrophic lateral sclerosis, frontotemporal lobar degeneration, dementia with Lewy bodies, mild cognitive impairment, Huntington's disease, traumatic brain injury and stroke. The invention is defined in the claims.

### BACKGROUND ART

Neurotrophic factors cerebral dopamine neurotrophic factor (CDNF) and mesencephalic astrocyte-derived neurotrophic factor (MANF) are currently the most efficient proteins for the treatment degenerating dopamine neurons in the rat 6-OHDA model of Parkinson's disease (Lindholm and Saarma, 2010). Both factors potently prevent the 6-OHDA-induced behavioral and histological symptoms of Parkinson's disease when applied before the toxin (Lindholm et al., 2007; Voutilainen et al., 2009). More importantly, posttreatment with either factor efficiently restored the normal motor behavior and dopaminergic innervations of the striatum when applied at the stage when the 6-OHDA-induced symptoms of the Parkinson's disease are already far-reaching (Lindholm et al., 2007; Voutilainen et al., 2011). CDNF protects and repairs dopamine neurons also in mouse MPTP model of Parkinson's disease (Airavaara et al., 2012), and in a severe 6-OHDA model it is more efficient than glial cell line-derived neurotrophic factor (GDNF)( Airavaara et al., 2012; Voutilainen et al 2011). The mechanisms behind the neuronal protection for these factors are not fully clear but it has been suggested they activate pathways, which aim at alleviating oxidative- and ER stress and depressing apoptotic cell death. Many pathophysiological conditions including diabetes mellitus and neurodegenerative diseases such as Parkinson's disease, Alzheimer's disease (AD) and amyotrophic lateral sclerosis (ALS) are associated with ER stress. Accordingly, the effect of CDNF and MANF has been shown in various central nervous system diseases (WO2009133247; WO2007068803; and Airavaara et al, 2009). In addition, CDNF and MANF suppress neuroinflammation, which is involved in the pathophysiology of most if not all CNS diseases and injuries (Nadella et al, 2014; Zhao et al, 2013).

In WO2014066686, a convection-enhanced delivery of MANF to a brain region of a patient is disclosed. A neurosurgical device for this invasive method comprises a catheter that can be placed at or near the brain region and a pump for generating a positive pressure gradient between the catheter and the brain region.

Aly et al., 2015, reviews current progress in intranasal gene delivery methods for treating Parkinson's disease. In the abstract, it is stated that intranasal delivery provides a promising approach for development of a non-invasive gene therapy for Parkinson's disease. The authors also discuss that CDNF and MANF have demonstrated efficacy in animal models of Parkinson's disease but have not yet been tested by the intranasal route. However, it is notable that nose-to-brain penetration properties of viral particles used in gene delivery methods are not dependent on the gene sequence of the chosen neurotrophic factor. In contrast, the intranasal penetration ability of a high molecular weight protein is always a unique and unpredictable property.

US6180603 discloses a method and a pharmaceutical composition for transporting a neurologic agent to brain in which the pharmaceutical composition is administered intranasally. The document specifically discloses pharmaceutical compositions comprising the neurotrophic factor NGF.

Alcalá-Barraza et al., 2010, disclose the intranasal delivery of BDNF, CNTF, neurotrophin-4/5 (NT-4/5), and erythropoietin (EPO), resulting in nanomolar concentrations of the molecules in rat brain. In a corresponding way, Bender et al.,2015, disclose intranasal administration of GDNF, either in liposomal formulation or in PBS, to rat brain.

Pardeshi & Shailendra (2013) disclose that conventional routes of drug administration, such as oral administration, are not able to deliver a number of therapeutic agents to the brain efficiently. The blood-brain barrier (BBB) and blood-cerebrospinal fluid barrier (BCB) hinder the entrance of drugs from systemic circulation into the central nervous system (CNS). The authors review direct nose-to-brain-drug-delivery and conclude that a variety of neurotherapeutic agents can be delivered to the CNS by nasal route. However, one of major limitations of the intranasal delivery as listed by the authors is decreased permeability of high molecular weight drugs across nasal mucosa.

### SUMMARY OF THE INVENTION

It is an aim of the present invention to provide compositions for intranasal administration for use in the intranasal treatment of central nervous system diseases.

It is another aim of the disclosure to provide novel uses of CDNF and MANF polypeptides.

In the present disclosure, it has been discovered that surprisingly chemical and biological characteristics of CDNF and MANF polypeptides facilitate the transport of the polypeptides to the central nervous system (CNS) when the polypeptides are intranasally administered to a patient. CDNF and MANF polypeptides are therefore able to bypass blood-brain-barrier through nasal route and reach the CNS.

Thus, in one aspect the invention provides a composition for intranasal administration comprising a CDNF polypeptide comprising the amino acid sequence of SEQ ID NO:1 or a functional fragment thereof for use in the intranasal treatment of a central nervous system disease.

In another aspect, the disclosure provides a composition for intranasal administration comprising a MANF polypeptide comprising the amino acid sequence of SEQ ID NO:3 or a functional fragment thereof for use in the intranasal treatment of a central nervous system disease.

### BRIEF DESCRIPTION OF THE DRAWINGS

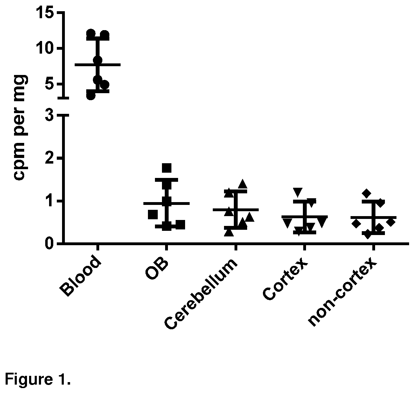

**Figure 1****.** Brain and plasma distribution of intranasally administered ¹²⁵I-labeled recombinant human (rh)CDNF in unlesioned Sprague Dawley rats. Approximately 10 ng of ¹²⁵I-labeled rhCDNF in 1% bovine serum albumin was administered intranasally into 6 rats under anesthesia. 20 min - 4 hours later blood samples were collected together with brain regions olfactory bulb, cerebellum, cortex and non-cortical areas. In total blood, 2.9 ± 1.3% of ¹²⁵I-labeled rhCDNF was detected in relation to total amount of delivered. The CPM values with subtracted background for brain tissues were 7-13% compared to the blood values indicating that between 0.16-0.36% of ¹²⁵I-labeled rhCDNF had reached brain tissues.

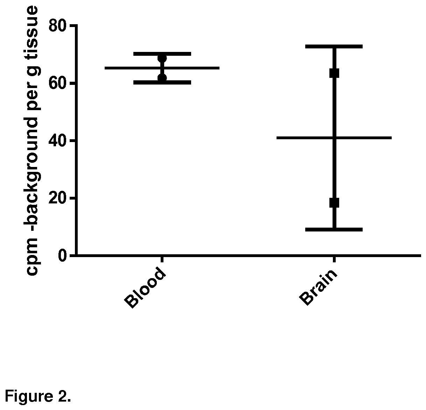

**Figure 2****.** Brain and plasma distribution of intranasally administered ¹²⁵I-labeled rhMANF in rats. 0.14-0.16 % of rhMANF reached the circulation. Male Sprague Dawley rats were anesthetized and went through middle cerebral artery occlusion (MCAO) for 60 min. Two days later rats were again anesthetized and they were administered approximately 8 ng ¹²⁵I-rhMANF (16 µl) together with 20 µg unlabelled rhMANF (in 4 µl). One hour later blood samples were collected and rats were perfused transcardially with saline and brains were collected. Results are expressed as counts per minute per milligram of wet tissue weight.

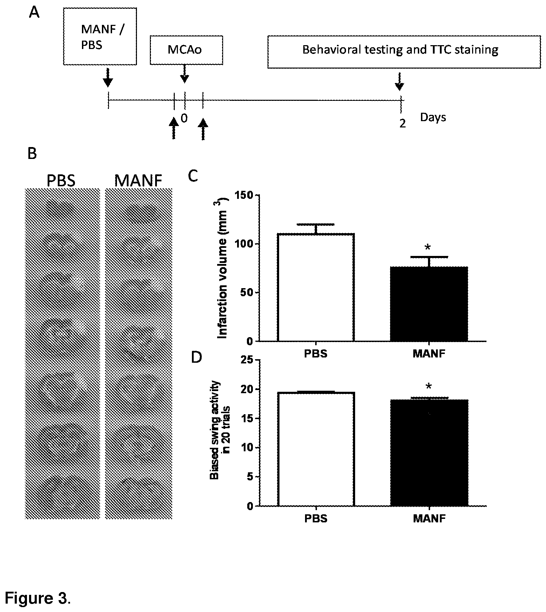

**Figure 3****.** Intranasally administered rhMANF reduces infarct volume in the MCAO rat model of ischemic stroke. (A) Timeline of the study. Rats were briefly anesthetized with isoflurane on the previous night. 10 µl of PBS or MANF solution was administered into each nostril in approximately 70° angle. On the next day, rats were anesthetized with chloral hydrate (400 mg/kg) and intranasal administration was repeated just before the MCAO and right after reperfusion. Middle cerebral artery together with the common carotid arteries were occluded for 60 minutes. Animals were necropsized 48 h post MCAO. (B) Example scans of triphenyltetrazolium chloride (TTC) staining of coronal brain sections 48 h after lesioning of PBS and rhMANF treated rats. (C) Infarction volumes measured by TTC staining. rhMANF reduced infarction volume as compared to the PBS-treated animals (P=0.034, Student's t-test). (D) Biased swing activity of PBS and rhMANF-treated animals. The neuroprotective effect was observed also as decreased body asymmetry (P=0.041, Student's t-test). In panels C and D, the PBS group consists of total of 17 rats and the MANF group consists of total of 24 rats. MANF group is combined from two rhMANF doses: dose of total of 20 µg (3.3 µg per nostril; 0.33 µg/µl solution n=10) and dose of total 60 µg (10 µg per nostril; 1 µg/µl solution, n=14). The MANF groups were combined here because the two dose groups showed very similar effect level.

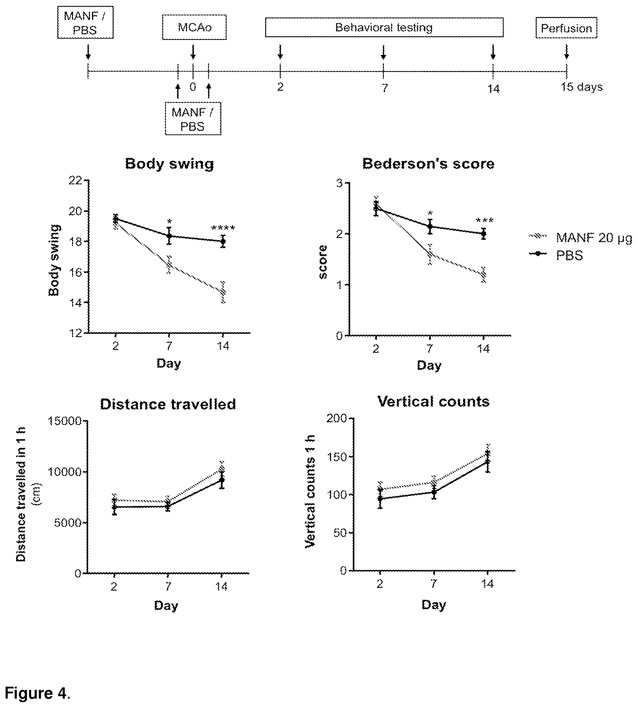

**Figure 4****.** Intranasally administered rhMANF promotes functional recovery in the MCAO rat model of ischemic stroke. 20 µg of rhMANF (10 µl per nostril; 0.33 µg/µl solution in PBS) or equal volume of PBS was administered intranasally under isoflurane anesthesia. (A) Study design. Intranasal administrations were done on the evening before the surgery, just before the MCAO and after reperfusion. Behavioral assessments were performed 2, 7 and 14 days after the MCAO and included body swing test (B), global neurological assessment (Bederson's score, C), distance travelled (D), and vertical activity (E) in a locomotor test. Data was analyzed using repeated measures ANOVA with Sidak's post hoc test (n=14-15). The two-way repeated measures ANOVA showed significant Treatment X Time interaction (F2, 54) = 9.34, P<0.001 for the body swing and F(2,54) =7.97, P<0.001 for the Bederson's score.

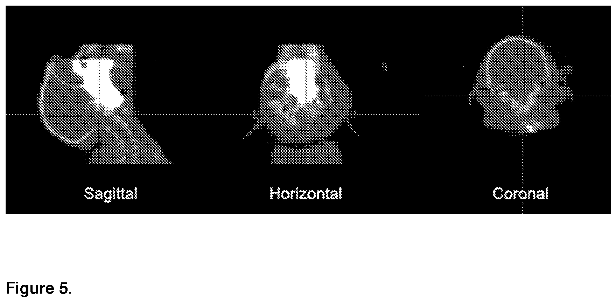

**Figure 5****.** Representative positron emission tomography (PET) - computed tomography (CT) images of a cynomolgus macaque dosed intranasally with ¹²⁴I-rhCDNF. 500 µCi of ¹²⁴I-labeled recombinant human rhCDNF was dosed per animal (n=4). Approximately 30% of the injected dose was located in the nasal cavity immediately after dosing.

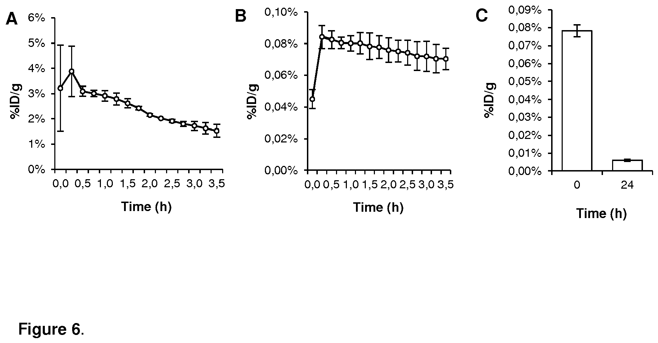

**Figure 6****.** Percent (%) injected dose per gram tissue (%ID/g) in upper nasal cavity (A) and whole brain (B) determined by dynamic focused brain PET scans between 0 and 3.5 h after intranasal dosing. Panel C shows that more than 90% of the brain-penetrated ¹²⁴I-rhCDNF is cleared from the brain within 24 hours from intranasal dosing.

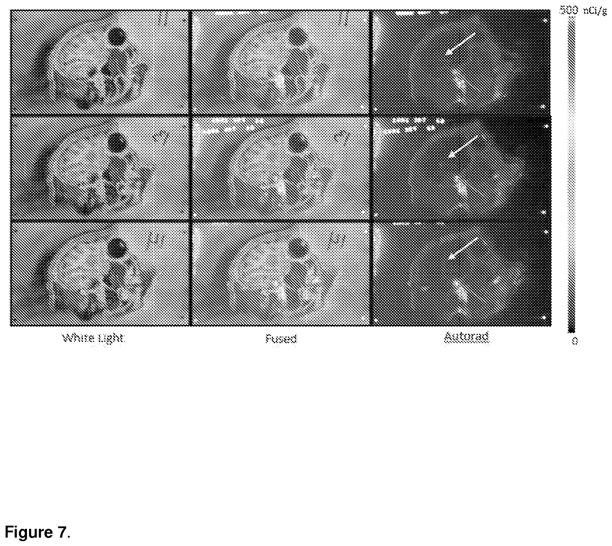

**Figure 7****.** Multi-view images combining white light images (left), co-registered white light and autoradioluminogram (middle) and autoradioluminogram only (right) of cynomolgus macaque brains after intranasal dosing of ¹²⁴I-rhCDNF. Signal seen in the lateral ventricles and the middle ear are indicated by arrows.

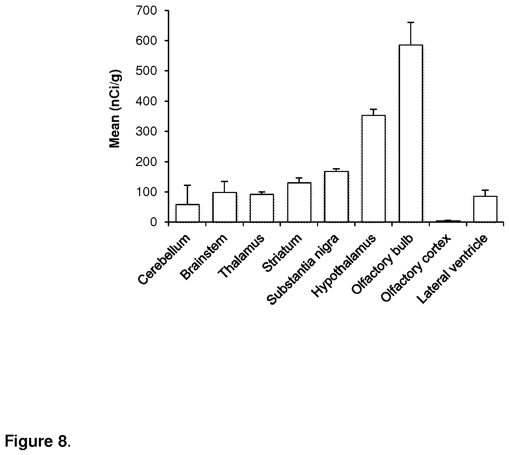

**Figure 8****.** Quantification of autoradiography data in Regions of Interest (ROI). The concentrations of radioactivity, derived from brain-penetrated ¹²⁴I-rhCDNF, are expressed as nCi per gram tissue (nCi/g).

### DESCRIPTION OF EMBODIMENTS

As used herein, a "polypeptide" generally refers to a molecule having an amino acid sequence encoded by a polynucleotide. "Polypeptide" also refers to a polymer composed of amino acid residues, related naturally occurring structural variants, and synthetic nonnaturally occurring analogs thereof linked via peptide bonds, related naturally occurring structural variants, and synthetic non-naturally occurring analogs thereof. Synthetic polypeptides can be synthesized, for example, using an automated polypeptide synthesizer.

As used herein, the term "fragment" as applied to a polypeptide, may ordinarily be at least about seven contiguous amino acids, typically, at least about fifteen contiguous amino acids, more typically, at least about thirty contiguous amino acids, typically at least about forty contiguous amino acids, preferably at least about fifty amino acids, even more preferably at least about sixty amino acids and most preferably the peptide fragment will be greater than about seventy contiguous amino acids in length. The term "functional fragment" refers to a fragment, which still retains biological activity of the intact polypeptide.

The present invention is related to a neurotrophic factor protein CDNF. CDNF polypeptides are the full-length human CDNF with a signal peptide having the total length of 187 amino acids and the mature human CDNF without the signal peptide having the total length of 161 amino acids (see Table 1) with the size of 18,5 kDa.

The present disclosure is also related to neurotrophic factor protein MANF. Particularly important MANF polypeptides are the full-length human MANF with a signal peptide having the total length of 179 amino acids and the mature human MANF without the signal peptide having the total length of 158 amino acids (see Table 1) with the size of 18 kDa. The MANF nucleic acid encoding mature MANF polypeptide without a signal peptide may be combined with a nucleic acid encoding another signal peptide to provide a full-length MANF polypeptide with a different signal peptide than the native MANF signal peptide.

Functional fragments of the MANF and CDNF polypeptides are also available for a skilled person as the production of fragments of whole polypeptides is known in the art. Accordingly, the MANF or CDNF protein may be a full length, naturally occurring form or may be a truncated or otherwise derivatised form. Examples of functional MANF or CDNF fragments are disclosed in WO2013034805, such as peptides comprising the sequence CKGC (SEQ ID NO:5) or CRAC (SEQ ID NO:6), preferably with the length of 4 - 40 amino acids. Further, Hellmann et al., 2011, disclose an active C-terminal fragment construct of MANF comprising residues 96-158 of SEQ ID NO:3.

In addition to naturally occurring allelic variants of MANF/CDNF, changes can be introduced by mutation into MANF/CDNF sequences that incur alterations in the amino acid sequences of the encoded MANF/CDNF polypeptide. Nucleotide substitutions leading to amino acid substitutions at "non-essential" amino acid residues can be made in the sequence of a MANF/CDNF polypeptide. MANF/CDNF polypeptides or functional fragments thereof comprising one or more "non-essential" substitutions can be seen as equivalents to wild-type MANF/CDNF polypeptides disclosed herein. These equivalents are preferably 90 %, more preferably 95 %, 96 %, 97 %, 98 % or 99 % homologous to said wild-type polypeptides.

A "non-essential" amino acid residue is a residue that can be modified in the wild-type sequences of MANF/CDNF without altering its biological activity, whereas an "essential" amino acid residue is required for such biological activity. For example, amino acid residues that are conserved among the MANF/CDNF molecules of the invention are predicted to be particularly non-amenable to alteration. Amino acids for which conservative substitutions can be made are well known in the art.

As discussed above, it has been found that CDNF and MANF polypeptides are able to bypass the blood-brain-barrier through the nasal route and to reach the central nervous system, CNS. Therefore, CDNF and MANF polypeptides can be intranasally administered to a patient and used for treatment of a number of central nervous system diseases.

Accordingly, in one embodiment a composition is provided for intranasal administration comprising a CDNF polypeptide comprising the amino acid sequence of SEQ ID NO:1 or a functional fragment thereof for use in the intranasal treatment of a central nervous system disease. Particularly, in this embodiment, intranasal treatment of Alzheimer's disease, Parkinson's disease, multiple system atrophy, amyotrophic lateral sclerosis, frontotemporal lobar degeneration, dementia with Lewy bodies, mild cognitive impairment, Huntington's disease, traumatic brain injury and stroke is achieved.

A second embodiment provides for the use of a composition comprising a CDNF polypeptide comprising the amino acid sequence of SEQ ID NO:1 or a functional fragment thereof for the manufacture of a medicament for use in the intranasal treatment of a central nervous system disease.

Another embodiment is a method for intranasal administration of a CDNF polypeptide to a subject suffering from a central nervous system disease, the method comprising the step of: intranasally administering to the subject in one or more doses a composition comprising a CDNF polypeptide comprising the amino acid sequence of SEQ ID NO:1 or a functional fragment thereof.

An embodiment is directed to a composition for intranasal administration comprising a MANF polypeptide comprising the amino acid sequence of SEQ ID NO:3 or a functional fragment thereof for use in the intranasal treatment of a central nervous system disease. Preferably, said central nervous system disease is selected from the group consisting of: Alzheimer's disease, Parkinson's disease, multiple system atrophy, amyotrophic lateral sclerosis, frontotemporal lobar degeneration, dementia with Lewy bodies, mild cognitive impairment, Huntington's disease, traumatic brain injury and stroke.

One further embodiment is the use of the composition comprising a MANF polypeptide comprising the amino acid sequence of SEQ ID NO:3 or a functional fragment thereof for the manufacture of a medicament for use in the intranasal treatment of a central nervous system disease.

Another further embodiment is a method for intranasal administration of a MANF polypeptide to a subject suffering from a central nervous system disease, the method comprising the step of: intranasally administering to the subject in one or more doses a composition comprising a MANF polypeptide comprising the amino acid sequence of SEQ ID NO:3 or a functional fragment thereof.

### Compositions suitable for intranasal administration

Compositions suitable for intranasal administration comprising CDNF/MANF polypeptides as active agents can be in the form of a liquid suspension, a liquid dispersion, a powder, a liposome, an aqueous solution or combinations thereof. The composition may thus be dispensed intranasally as a powdered or liquid nasal spray, nose drops, a gel or ointment, through a tube or catheter, by syringe, by packtail, by pledget, or by submucosal infusion. The composition may be administered to the nasal cavity alone or in combination with other neurologically active agents. CDNF/MANF polypeptides may be combined with a carrier and/or other adjuvants to form a pharmaceutical composition. Preferably, the composition comprise absorption promoters and absorption modulator systems such as cyclopenta decalactones, alkylsaccharides, chitosan, low methylated pectin or polyglycol hydroxystearate esters. Lipophilic substances in the form of micelles may be added to the composition to enhance absorption of the neurologic agent across the olfactory epithelium. Among those substances that are preferred micellar additives are GM-1 gangliosides and phosphatidylserine (PS), which may be combined with the neurologic agent either alone or in combination. Said absorption promoters (i.e. absorption enhancers) and absorption modulator systems may increase the transport of CDNF/MANF polypeptides across the nasal membrane and thus promote sufficiently high therapeutic levels in the CNS. It is believed that absorption promoters enhance drug delivery via the transcellular route or alterations in junctional proteins resulting in promotion of drug translocation through the paracellular route. Other absorption enhancers suitable for the present compositions are known in the art, see e.g. Stolnik et al., 2009 and Shubber et al., 2014.

Recombinant MANF and CDNF polypeptides for the compositions can be produced as disclosed in WO2009133247 and WO2007068803. For intranasal delivery purified recombinant protein can simply be added to a pharmaceutically suitable buffer. For instance, in Hanson et al, 2012, a growth factor was administered intranasally in acidic buffer comprising 20 mM sodium acetate at pH 4.25. In a preferred embodiment, the proteins are freeze-dried or spray-dried to produce a powder for use in a nasal spray. The methods for freeze-drying are well-known in the art (see e.g. Tang and Pikal 2004).

The optimal concentration of the active CDNF/MANF polypeptide in the composition will necessarily depend upon the characteristics of the patient, and the nature of the disease or condition for which the treatment is to be used. Preferably, the composition is administered intranasally 1-5 times a day, more preferably 1-2 times a day, and the amount of CDNF/MANF polypeptide is between 10 -1000 mg, more preferably 100-500 mg per dosage. The most acceptable dosing regimen would be one dose into one or both nostrils.

### Device

One example of suitable devices for intranasal administration is disclosed in WO2012119153. A suitable device is designed to deliver a drug in the form of a liquid suspension, a liquid dispersion, a powder, a liposome, an aqueous solution or combinations thereof. The device is preferably designed so that the administered nasal composition is delivered to the olfactory area in the upper third of the nasal cavity and particularly to the olfactory epithelium in order to promote transport of the active agents of the composition into the peripheral olfactory neurons. The device usually comprises a bottle, pump and actuator. Preferably, the device has a canister capable of containing a propellant, a diffuser in communication with the canister, a compound chamber for the drug in communication with the diffuser, and a nozzle in communication with the compound chamber. Devices are available for single dose or multiple dose systems. The powder formulations for the use in said device are preferably in aerosolised form. Although particular embodiments have been disclosed herein in detail, this has been done by way of example for purposes of illustration only. In particular, it is contemplated by the inventors that various substitutions, alterations, and modifications may be made. For instance, the choice of protocols is believed to be a matter of routine for a person of ordinary skill in the art with knowledge of the embodiments described herein.

Having generally described the invention above, the same will be more readily understood by reference to the following Experimental Section, which is provided by way of illustration and is not intended as limiting.

Although methods and materials similar or equivalent to those described herein can be used in the practice or testing of the present invention, suitable methods and materials are described below. The materials, methods, and examples are illustrative only and not intended to be limiting.

### EXAMPLES

### Example 1

The present study was designed to evaluate brain and plasma distribution of intranasally administered radiolabeled recombinant human CDNF protein (rhCDNF). ¹²⁵I-labelling of rhCDNF using the lactoperoxidase method and analysis were carried out as described in Voutilainen et al, 2011.

Animal use. ¹²⁵I-labelling of rhCDNF using the lactoperoxidase method and analysis were carried out as described in Voutilainen et al, 2011. Six unlesioned Sprague Dawley rats were anesthetized and approximately 10 ng of ¹²⁵I-labeled rhCDNF in 1% bovine serum albumin was administered intranasally under anesthesia. 20 min - 4 hours later blood samples were collected together with brain regions olfactory bulb, cerebellum, cortex and non-cortical areas.

Gamma counter analysis. Using gamma counting, 2.9 ± 1.3% (range 1.3-4.5%) of ¹²⁵I-labeled rhCDNF was found in blood (Figure 1). When the CPM values were compared to those found in the brain it was found that approximately 0.16-0.36% of ¹²⁵I-labeled rhCDNF had reached brain tissue. This data shows that rhCDNF protein, when administered to the nasal cavity in liquid form, can penetrate to the brain tissue in rats.

### Example 2

The present study was designed to evaluate brain and plasma distribution of intranasally administered radiolabeled recombinant human MANF protein (rhMANF).

Animal use. Two male Sprague Dawley rats were anesthetized and went through the procedure of middle cerebral artery occlusion (MCAO) together with common carotid artery occlusion for 60 min. Two days later rats were again anesthetized followed by intranasal administration of approximately 8 ng ¹²⁵I-rhMANF (16 µl), labeled using the lactoperoxidase method (Voutilainen et al, Exp. Neurol. 2011) and 20 µg of unlaballed rhMANF (in 4 µl). One hour later blood samples were collected and rats were perfused transcardially with saline and brains were collected.

Gamma-counter analysis. Approximately 0.14-0.16 % of ¹²⁵I-labeled rhMANF total dose was found in the brain and in the blood (Figure 2). This data shows that rhMANF protein, when administered to the nasal cavity in liquid form, can penetrate to the brain tissue in rats.

### Example 3

The present study was designed to evaluate therapeutic effects of intranasally administered recombinant human MANF protein (rhMANF).

Animal use, clinical and histopathological assessment. Rats were briefly anesthetized with isoflurane on the previous night. 10 µl of PBS or MANF solution was administered into each nostril in approximately 70° angle. On the next day, rats were anesthetized with chloral hydrate (400 mg/kg) and intranasal administration was repeated just before the MCAO and right after reperfusion. MCA together with common carotid arteries were occluded for 60 minutes. Animals were necropsized 48 h post MCAO.

Triphenyltetrazolium chloride (TTC) staining of coronal brain sections showed that MANF reduced infarction volume as compared with the PBS treated animals (P=0.034, Student's t-test, n=17-24; Figure 3). (D) The neuroprotective effect was observed also as decreased body asymmetry using the biased body swing test (P=0.041, Student's t-test). MANF group is combined from two MANF doses: dose of total of 20 µg (3.3 µg per nostril; 0.33 µg/µl solution n=10, average 68 ± 17) and dose of total 60 µg (10 µg per nostril; 1 µg/µl solution, n=14, average 80 ± 15), as both groups had highly similar outcomes.

In another set of animals, MANF dose of total of 20 µg (3.3 µg per nostril; 0.33 µg/µl) was administered in similar manner as above and behavior of rats was monitored over 2 weeks to measure the functional recovery. Biased swing activity, Bederson's score, distance travelled and vertical activity were analyzed on post stroke days 2, 7 and 14. The two-way repeated measures ANOVA showed significant Treatment X Time interaction (F2,54)=9.34, P<0.001 for the body swing and F(2,54)=7.97, P<0.001 for the Bederson's score (Figure 4; n=14-15). The data shows that pretreatment with intranasally administered recombinant MANF protein promoted functional recovery from stroke symptoms.

### Example 4

The present study was designed to evaluate brain penetration and kinetics of intranasally administered radiolabeled recombinant human CDNF protein (rhCDNF) in non-human primates. rhCDNF, labeled with ¹²⁴I using the lactoperoxidase method, was administered (400 µg, 500 µCi; formulated in 1% BSA, 0.1% sodium ascorbate and 0.1% gentisic acid, pH 6.4) to the nasal cavity of cynomolgus macaques. Positron emission tomographic (PET) imaging was used to determine nose-to-brain penetration of the ¹²⁴I-rhCDNF tracer.

Animal use. Four female cynomolgus (macaca fascicularis) monkeys (MPI Research) were used in this study. The monkeys were 2-4 years old and weighed 3.08 ± 0.22 kg on the day of imaging. Animal rooms were set to maintain a temperature of 18-26°C, a relative humidity of 50 ± 20%, both to the extent possible, and intermittent light and dark cycles of approximately 12 hours. Appropriate primate diet food and water ad libitum was provided. For various procedures where the animals were handled, e.g. physical examinations, the animals may have been sedated to effect with ketamine. Each animal was fasted for a minimum of 4 hours prior to induction of anesthesia with a ketamine dose (5-10 mg/kg IM), followed by isoflurane (1-2%) with oxygen (2%) for the duration of each scan. Animal welfare for this study was in compliance with the U.S. Department of Agriculture's (USDA) Animal Welfare Act (9 CFR Parts 1, 2, and 3). Guide for the Care and Use of Laboratory Animals, Institute of Laboratory Animal Resources, National Academy Press, Washington, D.C., 2011, was followed. The animal facility maintains an Animal Welfare Assurance statement with the National Institutes of Health Office of Laboratory Animal Welfare.

Device. A pressurized olfactory delivery device similar to that disclosed in U.S. Patent Application Publication No. US2012/027754 was used for intranasal delivery of rhCDNF.

Positron emission tomographic imaging and image analysis. Whole-body continuous bed motion and focused positron emission tomography (PET) and computed tomography (CT) data were acquired on a Focus220 microPET (Siemens Medical Systems, Knoxville, TN) and CereTom CT (NeuroLogica Corp, Danvers, MA), respectively. Each monkey was anaesthetized, intubated and placed on the PET bed in the head first and prone position for scanning. Two doses per nostril were administered on the bed. Immediately after the final dose had been administered a dynamic focused brain PET scan was acquired for 225 minutes. An additional scan was performed 24 h post-dosing to evaluate kinetics of brain clearance of the tracer.

Emission data were acquired in 3D list mode and rebinned into 2D sinograms. PET images were reconstructed by a 2D Ordered Subset Expectation Maximization (OSEM2D) algorithm. Corrections were made for detector normalization, decay, dead time, randoms, and attenuation. The dynamic scan was binned into 45 x 5 minute frames and 1 x 225 minute frame. Each reconstructed CT image was resampled and interpolated to the same voxel resolution as the accompanying PET image (0.9 mm). Each PET image was co-registered to the corresponding CT image (Figure 5).

Rotating Maximum Intensity Projection (MIP) movies and 3-slice view images centered on the brain were generated from the preprocessed PET/CT images. A linear color scale was chosen for the 0.2 to 20 %ID/mL PET intensity range. Regions of interest (ROIs) for the upper nasal cavity, throat and thyroid were defined by applying a combination of manual and automated segmentation thresholds to the PET or were hand drawn on the co-registered images. ROIs for various brain regions including the Entire Brain were acquired by fitting a proprietary 45-region Cynomolgus brain atlas to the brains of each monkey. A master spreadsheet was generated which included the volume, activity, and concentration (Activity/Volume) at each time point for each ROI generated.

The PET imaging data shows that recombinant human CDNF protein, as a drug substance, is capable of nose-to-brain penetration when aerosolized and delivered to the upper nasal cavity in healthy non-human primates (Figure 6A-B). Using a generic non-optimized formulation, intranasally delivered rhCDNF already reached brain levels corresponding to 1-4% of total injected dose. Brain areas with highest % injected dose per gram tissue amounts are listed in Table 2.

The PET imaging data also shows that approximately 92 ± 1% of brain-delivered rhCDNF is cleared from the brain within 24 hours (Figure 6C), suggesting that the brain half-life of rhCDNF is in the order of 6 hours. The use of *in vivo* imaging technique together with a device designed for nose-to-brain drug delivery in humans and large animals confirms that CDNF-family neurotrophic factors can be effectively delivered to the brain via the nasal administration route.

### Example 5

The present study was designed to evaluate brain penetration of intranasally administered radiolabeled recombinant human CDNF protein (rhCDNF). rhCDNF, labeled with ¹²⁴I using the lactoperoxidase method, was administered (400 µg, 500 µCi; formulated in 1% BSA, 0.1% sodium ascorbate and 0.1% gentisic acid, pH 6.4) to the nasal cavity of cynomolgus macaques. Autoradiography was used to determine nose-to-brain penetration of the ¹²¹I-rhCDNF tracer.

Animal use. The same animal procedures were used as in Example 4.

Device. The same intranasal dosing device and dosing procedure was used as in Example 4.

Autoradiography. At the scheduled termination time (after a 4-hour whole body imaging time point), one animal was euthanized and the head removed. The head was subsequently frozen in a hexane/dry ice bath and embedded in a 5% carboxymethylcellulose matrix. The embedded head was mounted onto a cryomacrotome stage maintained at approximately -10 to -30 °C and was sectioned to approximately 40 µm thick sections taken every 0.5 mm in the sagittal plane and captured on adhesive tape. Quantitative ¹²⁴I autoradiography was performed with 52 slices and 40 µm slice thickness in a section of the brain encompassing the area between both eyes.

In particular the following regions were analyzed: nasal cavity, olfactory bulb, olfactory tract, olfactory cortex, thalamus, striatum, substantia nigra, hypothalamus and lateral ventricle. A white light image of the block face was taken at each section. A set of sections were mounted, exposed to phosphor imaging screens and scanned using the Storm 860 image acquisition system (Figure 7). Quantification, relative to the calibration standards, was performed using the VivoQuant image analysis software. Calibration standard curve was constructed from the calculated activity in the autoradiography images and the normal concentrations of the [¹²⁴I]-calibration standards using linear regression. This was followed by image registration where the autoradiography slices were registered to the corresponding white light images. Then, registered images were loaded into VivoQuant image analysis software and ROIs were manually drawn on the white light data. Each ROI region was drawn on one representative slice and the mean intensity in the calibrated autoradiography images was calculated for each of them. The background radioactivity for each slice was reported as well for reference.

As shown in Figure 8, quantification of autoradiography data suggests that intranasally administered ¹²⁴I-rhCDNF penetrates to various brain structures of the cerebrum, cerebellum and the lateral ventricle.

To confirm the nose-to-brain delivery of rhCDNF in non-human primates, autoradiography was used to visualize radiolabeled rhCDNF in the sections of a non-human primate necropsized 4 hours after nasal administration of the tracer. The autoradiography data confirms that some labeled rhCDNF had penetrated to the brain from the upper nasal cavity. Signal was detected in various brain regions, including cerebellum, brainstem, thalamus, striatum, substantia nigra, hypothalamus and olfactory bulb. The presumed route of entry to lateral ventricles is via the cerebrospinal fluid (CSF) passages through the cribriform plate. These data suggest that intranasally administered rhCDNF gets access to the CSF allowing wide distribution in the CNS.

**Table 1. CDNF and MANF amino acid sequences.**

| |
|---|
| Mature CDNF amino acid sequence: |
| |
| CDNF amino acid sequence with a signal sequence: |
| |
| Mature MANF amino acid sequence: |
| |
| MANF amino acid sequence with a signal sequence: |
| |

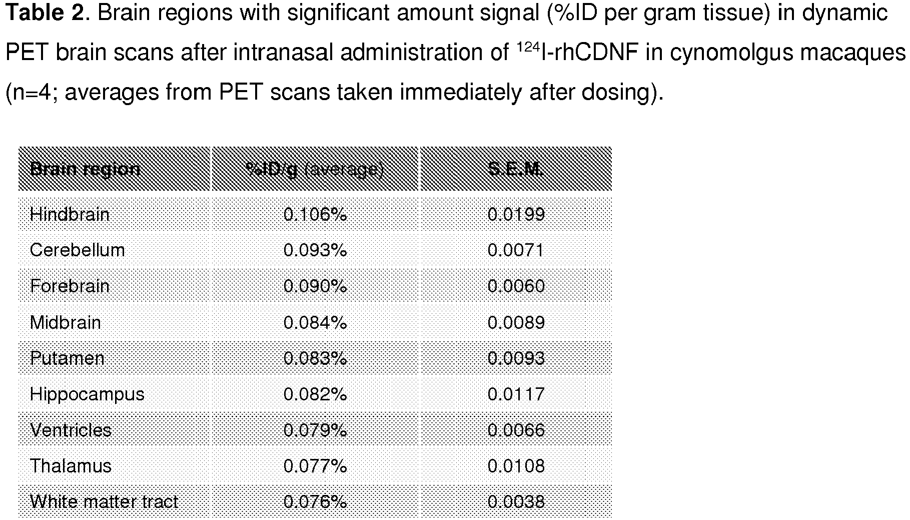

### CITATION LIST

### Patent Literature

US6180603
US2012/027754
WO2007068803
WO2009133247
WO2012119153
WO2014066686,

### Non-Patent Literature

Airavaara, M. et al. 2009. Mesencephalic astrocyte-derived neurotrophic factor reduces ischemic brain injury and promotes behavioral recovery in rats. J Comp Neurol 515 (1), 116.
Airavaara, M. Harvey, B.K. Voutilainen, M.H. Shen, H. Chou, J Lindholm, P. Lindahl, M. Tuominen. R.K. Saarma, M. Wang, Y., and B. Hoffer. 2012. CDNF protects the nigrostriatal dopamine system and promotes recovery after MPTP treatment in mice. Cell Transplant. 2012;21 (6):1213-23.
Alcalá-Barraza SR, Lee MS, Hanson LR, McDonald AA, Frey WH and McLoon LK. 2010. Intranasal delivery of neurotrophic factors BDNF, CNTF, EPO, and NT-4 to the CNS. Journal Of Drug Targeting 18(3):179-190.
Aly, AE-E and Waszczac, B. 2015. Intranasal gene delivery for treating Parkinson's disease: overcoming the blood-brain barrier, Expert Opin. Drug Deliv. 12(12):1923-1941.
Bender TS, Migliore MM, Campbell RB, John Gatley S and Waszczak BL. 2015. Intranasal administration of glial-derived neurotrophic factor (GDNF) rapidly and significantly increases whole-brain GDNF level in rats. Neuroscience, 303: 569-576.
Hanson, LR et al. 2012. Intranasal delivery of growth differentiation factor 5 to the central nervous system. Drug Deliv 19(3):149-154.
Hellman M, Arumäe U, Yu Ly, Lindholm P, Peranen J, Saarma M, Permi P (2011) Mesencephalic Astrocyte-derived Neurotrophic Factor (MANF) Has a Unique Mechanism to Rescue Apoptotic Neurons. J Biol Chem 286:2675-2680.
Lindholm, P., M.H. Voutilainen, J. Lauren, J. Peranen, V.M. Leppanen, J.O. Andressoo, M. Lindahl, S. Janhunen, N. Kalkkinen, T. Timmusk, R.K. Tuominen, and M. Saarma. 2007. Novel neurotrophic factor CDNF protects and rescues midbrain dopamine neurons in vivo. Nature. 448:73-77.
Lindholm, P., and M. Saarma. 2010. Novel CDNF/MANF family of neurotrophic factors. Dev. Neurobiol. 70:360-371.
Nadella R, Voutilainen MH, Saarma M, Gonzalez-Barrios JA, Leon-Chavez BA, Jiménez JM, Jiménez SH, Escobedo L, Martinez-Fong D. Transient transfection of human CDNF gene reduces the 6-hydroxydopamine-induced neuroinflammation in the rat substantia nigra. J. Neuroinflammation. 11: 209, 2014.
Pardeshi, CV and Belgamwar, VS. 2013. Direct nose to brain drug delivery via integrated nerve pathways bypassing the blood-brain barrier: an excellent platform for brain targeting. Expert Opin Drug Deliv 10(7):957-972.
Shubber S, Vllasaliu D, Rauch C, Jordan F, Illum, L, and Stolnik, S. 2015. Mechanism of Mucosal Permeability Enhancement of CriticalSorb® (Solutol® HS15) Investigated In Vitro in Cell Cultures. Pharm Res 32:516-527.
Stolnik S, Shakesheff K. Formulations for delivery of therapeutic proteins. Biotechnol Lett. 2009;31(1):1-11.
Tang, X and Pikal, MJ. 2004. Design of freeze-drying processes for pharmaceuticals: practical advice. Pharmaceutical Research 21(2):191-200.
Voutilainen, M.H., S. Back, J. Peranen, P. Lindholm, A. Raasmaja, P.T. Mannisto, M. Saarma, and R.K. Tuominen. 2011. Chronic infusion of CDNF prevents 6-OHDA-induced deficits in a rat model of Parkinson's disease. Exp. Neurol. 228:99-108.
Voutilainen, M.H., S. Back, E. Porsti, L. Toppinen, L. Lindgren, P. Lindholm, J. Peranen, M. Saarma, and R.K. Tuominen. 2009. Mesencephalic astrocyte-derived neurotrophic factor is neurorestorative in rat model of Parkinson's disease. J.Neurosci. 29:9651-9659. doi: 10.1523/JNEUROSCI.0833-09.2009.
Zhao H, Liu Y, Cheng L, Liu B, Zhang W, Guo YJ, Nie L. Mesencephalic astrocyte-derived neurotrophic factor inhibits oxygen-glucose deprivation-induced cell damage and inflammation by suppressing endoplasmic reticulum stress in rat primary astrocytes. J. Mol. Neurosci. 51(3): 671-8, 2013.

## Claims

1. A composition for intranasal administration comprising a CDNF polypeptide comprising the amino acid sequence of SEQ ID NO:1 or a functional fragment thereof for use in the intranasal treatment of a central nervous system disease.

2. The composition according to claim 1 for use in the intranasal treatment of a central nervous system disease, wherein said central nervous system disease is selected from the group consisting of: Alzheimer's disease, Parkinson's disease, multiple system atrophy, amyotrophic lateral sclerosis, frontotemporal lobar degeneration, dementia with Lewy bodies, mild cognitive impairment, Huntington's disease, traumatic brain injury and stroke.

3. The composition according to claim 1 or 2 for use in the intranasal treatment of a central nervous system disease, wherein said central nervous system disease is Alzheimer's disease.

4. The composition according to any one of claims 1-3 for use in the intranasal treatment of a central nervous system disease, wherein said central nervous system disease is amyotrophic lateral sclerosis.

5. The composition according to any one of claims 1-4 for use in the intranasal treatment of a central nervous system disease, wherein said central nervous system disease is Parkinson's disease.

6. The composition according to any one of claims 1-5 for use in the intranasal treatment of a central nervous system disease, wherein said central nervous system disease is stroke.

7. The composition according to any one of claims 1-6 for use in the intranasal treatment of a central nervous system disease, wherein said composition is a liquid or powder.

8. The composition according to claim 7 for use in the intranasal treatment of a central nervous system disease, wherein said composition is a liquid nasal spray or nose drops.

9. The composition according to claim 8 for use in the intranasal treatment of a central nervous system disease, wherein said composition is a liquid nasal spray with an absorption promoter.

10. The composition according to claim 7 for use in the intranasal treatment of a central nervous system disease, wherein said composition is a powder.

11. The composition according to any one of the preceding claims for use in the intranasal treatment of a central nervous system disease, wherein said CDNF polypeptide is the mature CDNF having amino acid sequence of SEQ ID NO:1.

## Patentansprüche

1. Zusammensetzung zur intranasalen Verabreichung, umfassend ein CDNF-Polypeptid, das die Aminosäuresequenz von SEQ ID Nr:1 oder ein funktionales Fragment davon umfasst, zum Gebrauch bei der intranasalen Behandlung einer Erkrankung des zentralen Nervensystems.

2. Zusammensetzung nach Anspruch 1 zum Gebrauch bei der intranasalen Behandlung einer Erkrankung des zentralen Nervensystems, wobei die Erkrankung des zentralen Nervensystems ausgewählt ist aus der Gruppe bestehend aus: Alzheimer-Krankheit, Parkinson-Krankheit, Multisystematrophie, amyotrophe Lateralsklerose, frontotemporale lobäre Degeneration, Lewy-Körper-Demenz, leichte kognitive Beeinträchtigung, Huntington-Krankheit, Schädel-Hirn-Trauma und Schlaganfall.

3. Zusammensetzung nach Anspruch 1 oder 2 zum Gebrauch bei der intranasalen Behandlung einer Erkrankung des zentralen Nervensystems, wobei die Erkrankung des zentralen Nervensystems Alzheimer-Krankheit ist.

4. Zusammensetzung nach einem der Ansprüche 1-3 zum Gebrauch bei der intranasalen Behandlung einer Erkrankung des zentralen Nervensystems, wobei die Erkrankung des zentralen Nervensystems amyotrophe Lateralsklerose ist.

5. Zusammensetzung nach einem der Ansprüche 1-4 zum Gebrauch bei der intranasalen Behandlung einer Erkrankung des zentralen Nervensystems, wobei die Erkrankung des zentralen Nervensystems Parkinson-Krankheit ist.

6. Zusammensetzung nach einem der Ansprüche 1-5 zum Gebrauch bei der intranasalen Behandlung einer Erkrankung des zentralen Nervensystems, wobei die Erkrankung des zentralen Nervensystems Schlaganfall ist.

7. Zusammensetzung nach einem der Ansprüche 1-6 zum Gebrauch bei der intranasalen Behandlung einer Erkrankung des zentralen Nervensystems, wobei die Zusammensetzung eine Flüssigkeit oder ein Pulver ist.

8. Zusammensetzung nach Anspruch 7 zum Gebrauch bei der intranasalen Behandlung einer Erkrankung des zentralen Nervensystems, wobei die Zusammensetzung ein flüssiges Nasenspray oder Nasentropfen ist.

9. Zusammensetzung nach Anspruch 8 zum Gebrauch bei der intranasalen Behandlung einer Erkrankung des zentralen Nervensystems, wobei die Zusammensetzung ein flüssiges Nasenspray mit einem Absorptionsförderer ist.

10. Zusammensetzung nach Anspruch 7 zum Gebrauch bei der intranasalen Behandlung einer Erkrankung des zentralen Nervensystems, wobei die Zusammensetzung ein Pulver ist.

11. Zusammensetzung nach einem der vorstehenden Ansprüche zum Gebrauch bei der intranasalen Behandlung einer Erkrankung des zentralen Nervensystems, wobei das CDNF-Polypeptid das reife CDNF ist, das Aminosäuresequenz von SEQ ID Nr:1 aufweist.

## Revendications

1. Composition pour administration intranasale comprenant un polypeptide CDNF comprenant la séquence d'acides aminés de SEQ ID NO : 1 ou un fragment fonctionnel de celle-ci pour son utilisation dans le traitement par voie intranasale d'une maladie du système nerveux central.

2. Composition selon la revendication 1 pour son utilisation dans le traitement par voie intranasale d'une maladie du système nerveux central, dans laquelle ladite maladie du système nerveux central est sélectionnée à partir du groupe consistant en : la maladie d'Alzheimer, la maladie de Parkinson, l'atrophie multisystématisée, la sclérose latérale amyotrophique, la dégénérescence lobaire frontotemporale, la démence à corps de Lewy, les déficiences cognitives légères, la maladie de Huntington, les lésions cérébrales traumatiques et les accidents vasculaires cérébraux.

3. Composition selon la revendication 1 ou 2 pour son utilisation dans le traitement par voie intranasale d'une maladie du système nerveux central, dans laquelle ladite maladie du système nerveux central est la maladie d'Alzheimer.

4. Composition selon l'une quelconque des revendications 1 à 3 pour son utilisation dans le traitement par voie intranasale d'une maladie du système nerveux central, dans laquelle ladite maladie du système nerveux central est la sclérose latérale amyotrophique.

5. Composition selon l'une quelconque des revendications 1 à 4 pour son utilisation dans le traitement par voie intranasale d'une maladie du système nerveux central, dans laquelle ladite maladie du système nerveux central est la maladie de Parkinson.

6. Composition selon l'une quelconque des revendications 1 à 5 pour son utilisation dans le traitement par voie intranasale d'une maladie du système nerveux central, dans laquelle ladite maladie du système nerveux central est un accident vasculaire cérébral.

7. Composition selon l'une quelconque des revendications 1 à 6 pour son utilisation dans le traitement par voie intranasale d'une maladie du système nerveux central, dans laquelle ladite composition est un liquide ou une poudre.

8. Composition selon la revendication 7 pour son utilisation dans le traitement par voie intranasale d'une maladie du système nerveux central, dans laquelle ladite composition est une pulvérisation nasale liquide ou des gouttes nasales.

9. Composition selon la revendication 8 pour son utilisation dans le traitement par voie intranasale d'une maladie du système nerveux central, dans laquelle ladite composition est une pulvérisation nasale liquide avec un promoteur d'absorption.

10. Composition selon la revendication 7 pour son utilisation dans le traitement par voie intranasale d'une maladie du système nerveux central, dans laquelle ladite composition est une poudre.

11. Composition selon l'une quelconque des revendications précédentes pour son utilisation dans le traitement par voie intranasale d'une maladie du système nerveux central, dans laquelle ledit polypeptide CDNF est le CDNF mature ayant la séquence d'acides aminés de SEQ ID NO : 1.
